Europäisches Patentamt

European Patent Office

Office européen des brevets

(11) Veröffentlichungsnummer: **0 514 636 A2**

# (12) EUROPÄISCHE PATENTANMELDUNG

(21) Anmeldenummer: **92104276.8**

(22) Anmeldetag: **12.03.92**

(51) Int. Cl.5: **A61L 2/06**, F24F 6/18

(30) Priorität: **15.03.91 DE 4108538**

(43) Veröffentlichungstag der Anmeldung:
**25.11.92 Patentblatt 92/48**

(84) Benannte Vertragsstaaten:
**AT BE CH DE DK ES FR GB GR IT LI LU NL PT SE**

(71) Anmelder: **Esser, Hans-Peter**
**Sonnenhang 10**
**W-5010 Bergheim 3(DE)**

(72) Erfinder: **Esser, Hans-Peter**
**Sonnenhang 10**
**W-5010 Bergheim 3(DE)**

(74) Vertreter: **Langmaack, Jürgen, Dipl.-Ing. et al**
**Patentanwälte Maxton . Maxton . Langmaack**
**Goltsteinstrasse 93 Postfach 51 08 06**
**W-5000 Köln 51(DE)**

(54) **Verfahren zur Bedampfung von Räumen und/oder zur thermischen Desinfektion von Oberflächen und Einrichtung zur Durchführung des Verfahrens.**

(57) Zur thermischen Desinfektion von Oberflächen, insbesondere abgeschlossenen durchströmbaren Räumen, wird anstelle eines Abwaschens oder Abspülens mit Flüssigkeit vorgeschlagen, daß ein aufgeheiztes nebelförmiges Medium erzeugt und mit den zu desinfizierenden Oberflächen in Kontakt gebracht wird. Hierdurch werden mit geringem Aufwand von Flüssigkeit schon nach kurzer Einwirkungszeit vorhandene Keime abgetötet.

EP 0 514 636 A2

Die Erfindung betrifft ein Verfahren zur Bedampfung von Räumen und/oder zur thermischen Desinfektion von Oberflächen, insbesondere abgeschlossenen durchströmbaren Räumen.

Die Desinfektion von Oberflächen kann in vielfältiger Weise vorgenommen werden. So ist es möglich, die Oberflächen mit Desinfektionsmittellösungen zu waschen oder abzuspülen, sofern die Oberflächen nicht zugänglich sind, wobei die Keime durch die chemische Einwirkung des Desinfektionsmittels abgetötet werden. Dieses Verfahren ist jedoch immer mit einem relativ hohen Wasserverbrauch verbunden, da in der Regel die Desinfektionsmittellösungen unter Verwendung von Wasser hergestellt werden oder aber die Oberflächen nachträglich mit Wasser abgespült werden müssen. Die Desinfektion von abgeschlossenen, insbesondere von außen nicht zugänglichen Räumen in Form von Kammern, Kanälen, Rohrleitungenssystem oder dergl. bereitet hierbei besondere Schwierigkeiten, zumal hier große Mengen an Desinfektionsmittellösungen eingesetzt werden müssen, die jedoch andererseits in ihrer Wirkung gar nicht voll ausgenutzt werden können.

Der Erfindung liegt nun die Aufgabe zugrunde, ein Verfahren zu schaffen, das mit einem möglichst geringem Flüssigkeitsaufwand, und auch für geschlossene unzugängliche Räume der vorstehend bezeichneten Art eine Desinfektion ermöglicht.

Diese Aufgabe wird gemäß der Erfindung dadurch gelöst, daß ein aufgeheiztes nebelförmiges Medium erzeugt und mit den zu desinfizierenden Oberflächen in Kontakt gebracht wird. Der Begriff "nebelförmiges Medium" im Sinne der vorliegenden Erfindung umfaßt sowohl einen mit Hilfe einer Flüssigkeit unter Termperatureinwirkung erzeugten Dampf, insbesondere einen Naßdampf oder oder einen Sattdampf, auch einen in einen Trägerstrom eingebrachten Naßdampf oder Sattdampf, als auch einen Nebel oder ein Aerosol, d. h. eine in ein aufgeheiztes Trägergas eingebrachte Flüssigkeit in feinster Verteilung. Überraschend hat sich nämlich gezeigt, daß beispielsweise ein mit Pseudomonas aeruginosa kontaminierte Rohrleitung nach einer Beaufschlagung mit Wasserdampf als nebelförmigem Medium mit einer Temperatur zwischen 70° und 90°C über einen Zeitraum von nur wenigen Minuten bakterienfrei war. Entsprechend einer vorteilhaften Ausgestaltung des erfindungsgemäßen Verfahrens wurde das aufgeheizte nebelförmige Medium als Strömung durch die zu desinfizierenden Räume hindurchgeführt. Der besondere Vorteil des erfindungsgemäßen Verfahrens besteht darin, daß im Vergleich zum Durchspülen mit einer Desinfektionsmittellösung erheblich weniger Flüssigkeit benötigt wird, wobei die Verwendung eines nebelförmigen Mediums auf der Basis von Wasser noch den Vorteil bietet, daß keinerlei Belastung der Atmosphäre durch Chemikalien erfolgt.

In einer vorteilhaften Ausgestaltung der Erfindung ist vorgesehen, daß das aufgeheizte nebelförmige Medium durch Verdampfen einer Flüssigkeit unter Temperatureinwirkung erzeugt wird. Dies hat insbesondere bei der Verwendung von Wasser den Vorteil, daß das in Form von Naßdampf oder Sattdampf vorliegende nebelförmige Medium eine hohe Temperaturlage besitzt, so daß eine einfache Einregulierung der je nach Einsatzfall erforderlichen Mindesttemperatur möglich ist. Die Regulierung kann beispielsweise über die Zumischung von zusätzlicher, ggf. aufgeheizter Trägerluft erfolgen. Die Verdampfung kann hierbei vorzugsweise unter Normaldruck erfolgen, wobei der beim Sieden der Flüssigkeit entstehende Dampfdruck ausreicht, das nebelförmige Medium durch bzw. in den zu beaufschlagenden und/oder den zu desinfizierenden Raum strömen zu lassen. Diese Verfahrensweise hat zudem den Vorteil, daß mit Dampfgeneratoren einfacher Bauart gearbeitet werden kann, die bei atmosphärischem Druck arbeiten und nicht als Dampfkessel im technischen Sinne ausgelegt sein müssen. Es ist aber auch möglich, den Verdampfungsvorgang unter Überdruck vorzunehmen und den so erzeugten Dampf in den Raum, beispielsweise eine Rohrleitung, einzuleiten, wobei der Dampf in reiner Form oder auch vermischt mit einem Trägergas, beispielsweise Luft, den Raum durchströmen kann. Der Vorteil der Erzeugung des nebelförmigen Mediums durch einen Verdampfungsvorgang, insbesondere durch eine Verdampfung von Wasser, hat den Vorteil, daß die eingesetzte Flüssigkeit durch den Verdampfungsvorgang selbst keimfreigemacht und dementsprechend durch die Flüssigkeit in den zu desinfizierenden Raum keine neuen Keime eingetragen werden.

In einer anderen Ausgestaltung des erfindungsgemäßen Verfahrens ist vorgesehen, daß zur Erzeugung des nebelförmigen Mediums eine Flüssigkeit in feinster Verteilung in ein vorzugsweise aufgeheiztes Trägergas zur Bildung eines Nebels oder Aerosols eingebracht wird. Diese Verfahrensweise hat den Vorteil, daß der für die Strömung erforderliche Volumenstrom im wesentlichen durch die Trägergasmenge bereitgestellt wird, während die für die Bedampfungswirkung erforderliche Flüssigkeitsmenge dosiert in diesen Trägergasstrom eingebracht wird. Die Verteilung der Flüssigkeit kann hierbei mechanisch über Düsen oder dergl. oder thermomechanisch über Blasenbildung erfolgen. Der Vorteil besteht hier auch darin, daß größere Strömungswiderstände überwunden werden können, aber auch Oberflächen gezielt "angestrahlt" werden können.

In Ausgestaltung der Erfindung ist hierbei vorgesehen, daß das Trägergas vor der Bildung des Aerosols aufgeheizt wird. Dies hat den Vorteil, daß große Mengen von Flüssigkeit vom Trägergas aufgenommen werden können, wobei eine Teilmenge erst nach einer gewissen Abkühlung des Trägergases im Verlaufe des Kontaktes mit den zu desinfizierenden Oberflächen in Tröpfchenform ausfällt.

In einer anderen Ausgestaltung des erfindungsgemäßen Verfahrens ist vorgesehen, daß das gebildete Aerosol vor dem Kontakt mit der zu desinfizierenden Oberfläche aufgeheizt wird. Hierdurch ist es möglich, die für die Desinfektion gewünschte Temperaturlage unmittelbar vor dem Einleiten des nebelförmigen Mediums in den zu bedampfenden und/oder zu desinfizierenden Raum einzustellen, wobei zugleich auch gewährleistet ist, daß die vom Trägergas mitgeführten Flüssigkeitströpfchen auch auf die geforderte Temperatur aufgeheizt werden.

In weiterer Ausgestaltung des erfindungsgemäßen Verfahrens ist vorgesehen, daß als Flüssigkeit zur Erzeugung des nebelförmigen Mediums, zumindest als Zusatz zu Wasser, ein temperaturbeständiges Desinfektionsmittel verwendet wird. Diese Ausgestaltung des Verfahrens hat den Vorteil, daß zusätzlich zu der thermischen Einwirkung auch noch eine chemische Einwirkung erfolgt. Dies ist insbesondere dann zweckmäßig, wenn die maximal zulässige Temperatur durch das Material der zu desinfizierenden Oberflächen beschränkt ist, beispielsweise eine Temperatur von 80°C nicht überschritten werden darf, gleichwohl damit zu rechnen ist, daß Keime vorhanden sind, die bei dieser Temperaturlage mit Sicherheit noch nicht abgetötet sind. Voraussetzung ist jedoch, daß das Desinfektionsmittel sich bei einer Erhitzung auf derartige Temperaturen chemisch nicht zersetzt. Weitere Voraussetzung ist, daß das verwendete Desinfektionsmittel in seiner Dampfform, auch vermischt mit Wasserdampf, beim Einatmen nicht gesundheitsschädlich ist.

In besonders vorteilhafter Ausgestaltung ist vorgesehen, daß die Flüssigkeit auf eine vorzugsweise temperaturregelbare Verdampferfläche aufgesprüht wird. Dies hat den Vorteil, daß zum einen der Trägergasstrom aufgeheizt werden kann und zum anderen die Verdampfungsleistung nicht vom Trägergasstrom sondern von der Verdampferfläche aufgebracht wird. Die vorherige Versprühung oder Zerstäubung der Flüssigkeit beschleunigt die Verdampfung und erlaubt eine feinfühlige Regulierung von Temperatur und "Nebelmenge".

Zur Überprüfung der Wirksamkeit des erfindungsgemäßen Verfahrens wurden in einem Versuch etwa 200 l handwarmes Leitungswasser mit 200 ml einer Pseudomonas aeruginosa-Kultur in einer Konzentration von $10^8$ Zellen/ml in einer fettigen Mischung mit Stearat und Pepton versetzt. Die Wassermenge wurde etwa 30 Minuten in einem mit Rohrleitungen versehenen Behältnis mit Luft durchwirbelt. Nach Ablaufen des Wasser wurden Tupferabstriche im Rohrsystem und im Bereich der Lufteinleitungsdüsen vorgenommen. Hierbei wurde in allen Bereichen der Wanne und der Rohrleitungen ein starkes Wachstum von Pseudomonas aeruginosa festgestellt.

Die von Hand nicht zugänglichen Rohrleitungen wurden anschliessend mit einem Wasserdampf unter Normaldruck mit einer Temperatur zwischen 100° und 90°C über mehrere Minuten bedampft. Anschließend wurden an den gleichen Stellen wie vor der Bedampfung Tupferabstriche vorgenommen. Diese ließen nach dem Bedampfen erkennen, daß in allen unmittelbar der Einwirkung des heißen Dampfes ausgesetzten Teilen keinerlei Keime trotz der relativ kurzen Einwirkungszeit mehr nachweisbar waren. Lediglich im Bereich von Rohrfittings konnte nach Lösen der Fittings noch eine geringe Restkontamination festgestellt werden, die jedoch bei längerer Einwirkungszeit ebenfalls beseitigt ist.

Die Erfindung betrifft ferner eine Einrichtung zur Erzeugung eines nebelförmigen Mediums, die gekennzeichnet ist durch ein mit einem Auslaß verbundenes Gehäuse, das mit einem Gebläse und mit wenigstens einer Heizeinrichtung zur Aufheizung von Gebläseluft versehen ist, und wenigstens einer Sprühvorrichtung zur Erzeugung feiner Flüssigkeitströpfchen, die in das Gehäuse im Bereich der Heizeinrichtung mündet. Besonders zweckmäßig ist es, wenn in weiterer Ausgestaltung der Sprühstrahl gegen eine Verdampferfläche der Heizeinrichtung gerichtet ist. Hierdurch wird erreicht, daß auch noch bei verhältnismäßig grober Zerstäubung eine schnelle Verdampfung erzielt wird, da die Verdampfung nach Art einer Filmverdampfung nach dem Kontakt mit der heißen Oberfläche erfolgt.

In besonders vorteilhafter Ausgestaltung ist hierbei vorgesehen, daß die Sprühvorrichtung als drucklos-mechanische Zerstäubervorrichtung, insbesondere als Schwingzerstäuber ausgebildet ist. Hierdurch ist eine gute Regelbarkeit der zuzuführenden Flüssigkeits- bzw. Tröpfchenmenge unabhängig vom Trägergasstrom ohne Beeinflussung des Zerstäubungsgrades gegeben.

In zweckmäßiger Ausgestaltung ist ferner vorgesehen, daß die Heizeinrichtung mit einem Temperaturfühler in Verbindung steht und daß im Bereich des Auslasses ein Temperaturfühler angeordnet ist und daß beide Temperaturfühler mit einer Regeleinrichtung zur Veränderung der Sprühmenge verbunden sind.

Die Erfindung wird anhand einer schematischen Zeichnung eines Ausführungsbeispieles näher erläutert:

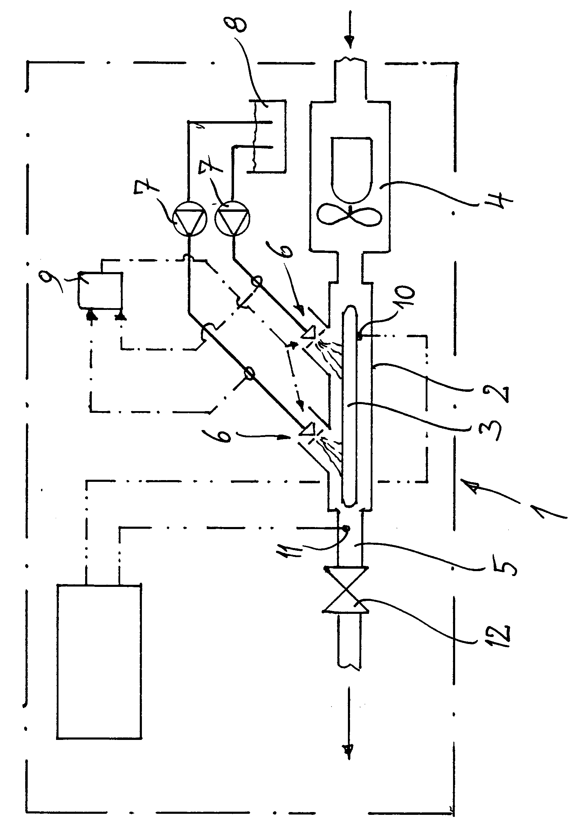

In der Figur ist eine spezielle Ausführungsform der Einrichtung 1 dargestellt. Diese weist eine Verdampferkammer 2 auf, in der eine beheizbare Verdampferfläche 3 angeordnet ist. In diese Verdampferkammer 2 mündet der Auslaß eines Gebläses 4 ein, so daß mit Luft als Trägergas die Verdampferkammer 2 durchströmt werden kann. Der über das Sperrventil 12 absperrbare Auslaß 5 der Verdampferkammer 2 ist an das zu bedampfende System.

In die Verdampferkammer 2 mündet wenigstens eine Sprüheinrichtung 6 ein, deren Sprühstrahl gegen die Verdampferfläche 3 gerichtet ist. Zur Vereinfachung einer Mengenregelung sind hier zwei Sprüheinrichtungen 6 angeordnet. Diese können entweder als Druckluftsprüheinrichtung, bevorzugt aber als drucklos-mechanische Sprüheinrichtung, beispielsweise als Schwingzerstäuber ausgebildet sein. Bei dieser Ausführung wird die zu zerstäubende Flüssigkeit aus einem höherliegenden Behälter oder, wie hier dargestellt, mittels Förderpumpe 7 aus einem Behälter 8 dem System zugeführt. Über einen Wächter 9, der mit der Energieversorgung des Schwingzerstäubers 6 in Verbindung steht, ist sichergestellt, daß dieser nur bei gefüllter Zuleitung eingeschaltet werden kann bzw. bei Wassermangel abschalten kann. Die Mengenregulierung kann bei einem Schwingzerstäuber unmittelbar über eine Verstellung der Amplitude des Schwingorgans erreicht werden.

Die Temperatur der Verdampferfläche 3 einerseits und des erzeugten nebelförmigen Mediums andererseits wird über Temperaturfühler 10 und 11 erfaßt und dient der Regelung der zuzuführenden Flüssigkeitsmenge und/oder der Temperatur der Verdampferfläche, so daß die gewünschte Temperatur eingehalten und/der eine Überhitzung ausgeschlossen werden kann. Falls eine höhere Temperatur im Bedampfungsbereich gewünscht wird, kann dies über eine entsprechende Aufheizung des Trägergases erreicht werden, wobei dann allerdings die eingesprühte Flüssigkeit nicht mehr als sichtbarer Nebel bzw. Dampf im physikalischen Sinn abgegeben wird.

Die beschriebene Einrichtung kann auch zur Bedampfung anderer sanitärer Anlagen verwendet werden. Bei entsprechender Auslegung der Heiz- und Verdampferleistung sowie der Zerstäuberleistung ist auch der Einsatz zur Bedampfung einer Dampfbadekabine möglich, da auch hier eine Grenze in der maximal zulässigen Temeratur einzuhalten ist.

**Patentansprüche**

1. Verfahren zur thermischen Bedampfung von Räumen und/oder zur Desinfektion von Oberflächen, insbesondere abgeschlossenen durchströmbaren Räumen,
   **dadurch gekennzeichnet**, daß ein aufgeheiztes nebelförmiges Medium erzeugt und mit den zu bedampfenden und/oder zu desinfizierenden Oberflächen in Kontakt gebracht wird.

2. Verfahren nach Anspruch 1, dadurch gekennzeichnet, daß das aufgeheizte nebelförmige Medium als Strömung durch die zu desinfizierenden Räume hindurchgeführt wird.

3. Verfahren nach Anspruch 1 oder 2, dadurch gekennzeichnet, daß das aufgeheizte nebelförmige Medium durch Verdampfen einer Flüssigkeit unter Temperatureinwirkung erzeugt wird.

4. Verfahren nach Anspruch 3, dadurch gekennzeichnet, daß der Verdampfungsvorgang unter normalem atmosphärischen Druck bewirkt wird.

5. Verfahren nach Anspruch 1 bis 4, dadurch gekennzeichnet, daß die Flüssigkeit auf eine vorzugsweise temperaturregelbare Verdampferfläche aufgesprüht wird.

6. Verfahren nach einem der Ansprüche 1 bis 5, dadurch gekennzeichnet, daß zur Erzeugung des nebelförmigen Mediums eine Flüssigkeit in feinster Verteilung zur Bildung eines Aerosols in ein Trägergas eingebracht wird.

7. Verfahren nach Anspruch 5 oder 6, dadurch gekennzeichnet, daß das Trägergas vor Bildung des Aerosols aufgeheizt ist.

8. Verfahren nach Anspruch 5, 6 oder 7, dadurch gekennzeichnet, daß das gebildete Aerosol vor dem Kontakt mit der zu desinfizierenden Oberfläche aufgeheizt wird.

9. Verfahren nach einem der Ansprüche 1 bis 8, dadurch gekennzeichnet, daß das nebelförmige Medium mit geringem Überdruck auf die zu desinfizierenden Oberfläche aufgebracht wird.

10. Verfahren nach einem der Ansprüche 1 bis 9, dadurch gekennzeichnet, daß als Flüssigkeit zur Erzeugung des nebelförmigen Mediums keimfreies Wasser verwendet wird.

11. Verfahren nach einem der Ansprüche 1 bis 10, dadurch gekennzeichnet, daß als Flüssigkeit zur Erzeugung des nebelförmigen Mediums zumindest als Zusatz zu Wasser ein temperaturbeständiges Desinfektionsmittel verwendet wird.

12. Verfahren nach einem der Ansprüche 1 bis 11, dadurch gekennzeichnet, daß die einzusprühende Flüssigkeitsmenge bei vorgegebener Temperatur des Trägergases und/oder der Heizleistung der Heizoberfläche in Abhängigkeit von der Temperatur des nebelförmigen Mediums hinter der Einsprühstelle geregelt wird.

13. Einrichtung zur Durchführung des Verfahrens nach den Ansprüchen 1 bis 12, gekennzeichnet durch ein an die Zuleitung angeschlossenes Gehäuse (2), das mit wenigstens einem Gebläse (4) und mit wenigstens einer Heizeinrichtung (3) zur Aufheizung von Gebläseluft in Verbindung steht, und wenigstens eine Sprühvorrichtung (6) zur Erzeugung feiner Flüssigkeitströpfchen, die in das Gehäuse (2) im Bereich der Heizeinrichtung (3) mündet.

14. Einrichtung nach Ansprüch 13, dadurch gekennzeichnet, daß der Sprühstrahl gegen eine Verdampferfläche der Heizeinrichtung (3) gerichtet ist.

15. Einrichtung nach Anspruch 13 oder 14, dadurch gekennzeichnet, daß die Sprühvorrichtung (6) als drucklosmechanische Zerstäubervorrichtung, insbesondere Schwingzerstäuber, ausgebildet ist.

16. Einrichtung nach einem der Ansprüche 12 bis 15, dadurch gekennzeichnet, daß zur Regelung der einzusprühenden Flüssigkeitsmenge und/oder der Temperatur die Heizeinrichtung (3) mit einem Temperaturfühler (10) in Verbindung steht und daß ein Temperaturfühler (11) im Bereich der Zuleitung (12) angeordnet ist und daß beide Temperaturfühler mit einer Regeleinrichtung zur Veränderung der Sprühflüssigkeitsmenge verbunden sind.